# EUROPEAN PATENT APPLICATION

(11) **EP 3 466 873 A1**
(43) Date of publication of application: **10.04.2019**
(21) Application number: 17802889.0
(22) Date of filing: 25.05.2017
(51) Int. Cl.: C01B 21/086, H01M 10/0568

(54) **METHOD FOR PRODUCING BIS(FLUOROSULFONYL)IMIDE ALKALI METAL SALT**

(30) Priority: 27.05.2016 JP 2016106033
(71) Applicant: Nippon Shokubai Co., Ltd., Osaka-shi, Osaka 541-0043 (JP)
(72) Inventor: ITAYAMA, Naohiko, Suita-shi Osaka 564-0034 (JP); OKUMURA, Yasunori, Suita-shi Osaka 564-0034 (JP); KATSUYAMA, Hiromoto, Suita-shi Osaka 564-0034 (JP); MIZUNO, Hiroyuki, Suita-shi Osaka 564-0034 (JP); FUKATA, Yukihiro, Suita-shi Osaka 564-0034 (JP)
(74) Representative: Müller-Boré & Partner Patentanwälte PartG mbB
(86) International application number: PCT/JP2017/019584
(87) International publication number: WO 2017/204303

(57) **Abstract**

Provided is a method with which it is possible to conveniently produce bis(fluorosulfonyl)imide suitable as a nonaqueous electrolyte of a lithium ion secondary cell. The method for producing a bis(fluorosulfonyl)imide alkali metal salt of the invention is a production method for producing a bis(fluorosulfonyl)imide alkali metal salt by reacting bis(fluorosulfonyl)imide and an alkali metal halide in a reaction solution including an organic solvent, the method including a purification step for filtering out the bis(fluorosulfonyl)imide alkali metal from the solution after the reaction.

## Description

### TECHNICAL FIELD

The present invention relates to a method for producing a bis(fluorosulfonyl)imide alkali metal salt.

### BACKGROUND ART

Bis(fluorosulfonyl)imide alkali metal salts are compounds that are useful in various applications as electrolytes for non-aqueous type electrolyte solutions (herein after may be referred to as non-aqueous electrolyte solution), as additives to electrolyte solutions of fuel cells, and as antistatic agents and the like. Particularly in recent years, alkali metal batteries, specifically lithium ion secondary batteries, due to its high energy density, are used as a power source for mobile communication terminals and for portable information terminals. The market of such batteries has increased rapidly with the spread of the terminals.

As a method for producing a bis(fluorosulfonyl)imide alkali metal salt, Patent Document 1 discloses a process for preparing a bis(fluorosulfonyl)imide salt by reacting bis(fluorosulfonyl)imide with lithium fluoride in acetonitrile, followed by removing a solid by centrifugation, and concentrating and drying a solution. Also, Patent Document 2 discloses a process for preparing a bis(fluorosulfonyl) imide salt by reacting bis(fluorosulfonyl) imide with lithium carbonate in an organic solvent, followed by removing a solid by filtering, and concentrating and drying a solution.

### PRIOR ART DOCUMENTS

### PATENT DOCUMENTS

Patent Document 1: Japanese Domestic Re-publication of PCT publication Hei8-511274
Patent Document 2: Japanese Domestic Re-publication of PCT publication 2015-536898

### SUMMARY OF INVENTION

### TECHNICAL PROBLEM

However, there are possibilities in Patent Document 1 or Patent Document 2 that an unreacted bis(fluorosulfonyl)imide or an organic solvent may remain in a bis(fluorosulfonyl)imide salt because the bis(fluorosulfonyl)imide lithium salt dissolved in the organic solvent is isolated from the solvent by evaporation to dryness. Also, there are possibilities in Patent Document 2 that a bis(fluorosulfonyl)imide lithium salt may be decomposed by water generated together with carbon dioxide gas from lithium carbonate used for obtaining a lithium salt.

Under these circumstances, the present invention has been made and an object thereof is to provide a method for easily producing a bis(fluorosulfonyl)imide alkali metal salt in high purity. A bis (fluorosulfonyl) imide alkali metal salt obtained by the production method of the present invention is suitably used for non-aqueous electrolytic solutions such as lithium ion secondary batteries.

### SOLUTIONS TO THE PROBLEMS

The present invention is a method for producing a bis(fluorosulfonyl)imide alkali metal salt, comprising:
a step for producing a bis(fluorosulfonyl)imide alkali metal salt by reacting a bis(fluorosulfonyl)imide with an alkali metal halide in a reaction solution including an organic solvent, and
a purification step for filtering the bis(fluorosulfonyl)imide alkali metal salt from the reaction solution after the reaction.

The organic solvent preferably includes a poor solvent for the bis(fluorosulfonyl)imide alkali metal salt, wherein the poor solvent is at least one selected from the group consisting of an aromatic hydrocarbon-based solvent (including halogenated hydrocarbon), an alphatic hydrocarbon-based solvent (including halogenated hydrocarbon), and an aromatic ether-based solvent.

A ratio of the poor solvent in the organic solvent is preferably 70% by weight or more.

The organic solvent is preferably at least one selected from the group consisting of an aromatic hydrocarbon-based solvent (including halogenated hydrocarbon), an alphatic hydrocarbon-based solvent (including halogenated hydrocarbon), and an aromatic ether-based solvent. Particularly, the organic solvent is preferably a poor solvent for the bis(fluorosulfonyl)imide alkali metal salt, wherein the poor solvent is preferably at least one selected from the group consisting of an aromatic hydrocarbon-based solvent (including halogenated hydrocarbon), an alphatic hydrocarbon-based solvent (including halogenated hydrocarbon), and an aromatic ether-based solvent.

The poor solvent is preferably at least one selected from the group consisting of toluene, o-xylene, m-xylene, p-xylene, ethylbenzene, isopropylbenzene, 1,2,4-trimethylbenzene, hexane, heptane, chlorobenzene, dichlorobenzene, dichloromethane, 1,2-dichloroethane, anisole and cyclohexane.

A mole ratio of the alkali metal halide to the bis(fluorosulfonyl)imide is 1.00 or less.

The production method of the present invention is preferred that a generated hydrogen halide is removed from the reaction solution during the reaction.

The alkali metal halide is preferably LiF.

The filtering is preferably performed by using a filter medium having retained particle diameter of 0.1 to 10µm.

A washing for a filter residue is preferably operated by using a poor solvent for the bis(fluorosulfonyl) imide alkali metal salt after the filtering in the purification step for conducting the filtering, wherein
the poor solvent is at least one selected from the group consisting of an aromatic hydrocarbon-based solvent (including halogenated hydrocarbon), an alphatic hydrocarbon-based solvent (including halogenated hydrocarbon), and an aromatic ether-based solvent.

### EFFECTS OF THE INVENTION

According to the present invention, a high-purity bis(fluorosulfonyl)imide alkali metal salt can be produced in a simple purification method that a bis(fluorosulfonyl)imide alkali metal salt precipitated in an organic solvent is isolated only by filtering and therefore the production costs can be reduced.

### DESCRIPTION OF EMBODIMENTS

Hereinafter, the present invention is described in more detail. In the following description, "%" is "% by mass", "part" is "part by mass" and a range of "A-B" is A or more and B or less unless otherwise noted.

The present invention is a method for producing a bis(fluorosulfonyl)imide alkali metal salt, comprising: a step for producing a bis(fluorosulfonyl)imide alkali metal salt by reacting a bis(fluorosulfonyl)imide with an alkali metal halide in a reaction solution including an organic solvent, and a purification step for filtering the bis(fluorosulfonyl)imide alkali metal salt from the reaction solution after the reaction. Hereinafter, the reaction solution to be subjected to the purification process after completion of the reaction is referred to as "reaction solution after the reaction" or "solution after the reaction" to differentiate from a reaction solution during a reaction of or in a mixed stage of bis (fluorosulfonyl)imide, an alkali metal halide and an organic solvent.

The bis(fluorosulfonyl)imide alkali metal salt includes lithium bis(fluorosulfonyl)imide (LiFSI), sodium bis(fluorosulfonyl)imide (NaFSI), potassium bis(fluorosulfonyl)imide (KFSI) and the like. Among these examples, lithium bis(fluorosulfonyl)imide is preferred.

### [Reaction between bis(fluorosulfonyl)imide alkali metal salt and an alkali metal halide]

A reaction between bis(fluorosulfonyl)imide (HFSI) and an alkali metal halide is conducted in a reaction solution containing an organic solvent.

### [Bis (fluorosulfonyl)imide]

Bis(fluorosulfonyl)imide can be synthesized by conventionally-known methods. For example, bis(fluorosulfonyl)imide can be synthesized from a bis(sulfonyl halide)imide by using a fluorinating agent. Examples of the halogen in bis(sulfonyl halide)imide include Cl, Br, I and At other than F.

Hereinafter, a fluorination step in which bis(fluorosulfonyl)imide is synthesized from the bis(sulfonyl halide)imide by using the fluorinating agent is described. For example, a fluorination reaction of the bis(sulfonyl halide)imide may be carried out. Specifically, methods disclosed in CA2527802A, Jean'ne m. Shreeve et al., Inorg. Chem. 1998, 37(24), 6295-6303 are exemplified. And the bis(sulfonyl halide)imide to be used as a starting material may be a commercially available product, or may be synthesized by a known method. Also, bis(fluorosulfonyl)imide may be synthesized by using urea and fluorosulfonic acid as disclosed in Japanese Domestic Re-publication of PCT publication Hei8-511274.

### [Alkali metal halide]

The alkali metal halide in the production method of the present invention include: chlorides such as LiCl, NaCl, KCl, RbCl, and CsCl; fluorides such as LiF, NaF, KF, RbF and CsF. Among these examples, LiCl and/or LiF is most preferable. When the alkali metal halide is LiCl and/or LiF, the purification of the bis(fluorosulfonyl)imide alkali metal salt becomes easy because the boiling points of HCl and HF as by-products generated in the reaction of bis(fluorosulfonyl)imide and the alkali metal salt are low. Also, when Li is used for the metal in lithium ion secondary batteries, superior battery properties are achieved. Among examples, LiF is particularly preferable as explained below.

The mole ratio of the alkali metal halide to the bis(fluorosulfonyl)imide in the reaction between bis(fluorosulfonyl)imide and the alkali metal halide is preferably 1.00 or less. The upper limit of the mole ratio is, for examples, 0.99 or less, 0.98 or less and 0.95 or less. And the lower limit of the mole ratio is, for examples, 0.70 or more, 0.80 or more and 0.90 or more. When the mole ratio of the alkali metal halide to the bis(fluorosulfonyl)imide is included in the above range, an equivalent or less of the alkali metal halide is used for the reaction, it is possible to suppress a remaining unreacted alkali metal halide in a solid state. That is, even though the alkali metal halide is insoluble in the reaction solution, the mole ratio of the alkali metal halide to the bis(fluorosulfonyl)imide, particularly when the mole ratio of the alkali metal contained thereof, is included in this range and reaction conditions such as the reaction temperature is appropriately set as described later, then it is considered that the alkali metal halide is hardly any left after the reaction and thereby the removal operation of the alkali metal halide may possibly be simplified.

On the other hand, it is considered that the bis(fluorosulfonyl)imide alkali metal salt becomes an insoluble solid in the reaction solution and the bis(fluorosulfonyl)imide alkali metal salt is substantially only solid remained in the solution after the reaction which can be purified by the filtering. And the bis(fluorosulfonyl)imide in a liquid state allows to be removed by the filtering.

In the production method of the present invention, the amount of the bis (fluorosulfonyl) imide to be used in the reaction is preferably 5 to 95% by weight, more preferably 10 to 95% by weight relative to the total reaction solution. The lower limit of the bis (fluorosulfonyl) imide can set to 15% by weight or more. The upper limit of the bis (fluorosulfonyl) imide is preferably 90% by weight or lower, more preferably 85% by weight or lower, still more preferably 70% by weight or lower, particularly preferably 60% by weight or lower and most preferably 50% by weight or lower. When the amount of the bis (fluorosulfonyl) imide to the total reaction solution is included in the above range, the reaction proceeds to conduct purification step easily.

In the production method of the present invention, the amount of the alkali metal halide to be used in the reaction is preferably 0.1 to 35% by weight relative to the total reaction solution. The lower limit of the alkali metal halide is more preferably 0.5% by weight or more, still more preferably 1% by weight or more. The upper limit of is the alkali metal halide more preferably 30% by weight or lower, still more preferably 25% by weight or lower, even more preferably 20% by weight or lower, particularly preferably 15% by weight or lower and most preferably 10% by weight or lower. When the amount of the alkali metal compound to the total reaction solution is included in the above- range, the reaction proceeds to conduct purification step easily.

### [Organic solvent used for the reaction]

The organic solvent used in the production method of the present invention is not particularly limited, and a conventionally known organic solvent can be used. The organic solvent is preferably used as a solvent for the reaction between bis(fluorosulfonyl)imide and the alkali metal halide as an alkali metal compound and its purification. That is, the organic solvent, which is also referred to as a production solvent, is a solvent used preferably for the production of the bis(fluorosulfonyl)imide alkali metal salt. And the organic solvent, which is also referred to as a residual solvent, may remain in an electrolyte solution material or an electrolyte solution containing the bis(fluorosulfonyl)imide alkali metal salt.

According to the classification of the organic solvent on the basis of the affinity to the bis(fluorosulfonyl)imide alkali metal salt, a following good solvent and poor solvent are exemplified. The good solvent means a solvent which can dissolve the bis(fluorosulfonyl)imide alkali metal salt. On the other hand, the poor solvent means a solvent which shows insoluble or hardly soluble against the bis(fluorosulfonyl)imide alkali metal salt. It is noted that "hardly insoluble" means a solvent having solubility to the bis(fluorosulfonyl)imide alkali metal salt about 10000mg/L at 25°C.

The specific examples of the good solvent having a moderate level of affinity to the bis(fluorosulfonyl)imide alkali metal salt include: water; an alcohol- based solvent, such as methanol, ethanol, propanol and butanol; a carboxylic acid- based solvent, such as formic acid and acetic acid; a ketone, such as acetone, methyl ethyl ketone, methyl isobutyl ketone and diisobutyl ketone; a nitrile-based solvent, such as isobutyronitrile, acetonitrile, valeronitrile and benzonitrile; a chain ester-based solvent, such as ethyl acetate, isopropyl acetate and butyl acetate; a chain ether-based solvent having one oxygen atom within its molecule, such as diethyl ether, diisopropyl ether, t-butyl methyl ether and cyclopentyl methyl ether; a nitro-group-containing solvent, such as nitromethane and nitrobenzene; N-methylpyrrolidone; and a glyme-based solvent. Among these solvents, acetonitrile, valeronitrile, ethyl acetate, isopropyl acetate, butyl acetate and cyclopentyl methyl ether are preferred.

Specific examples of the poor solvent having low affinity to the bis(fluorosulfonyl)imide alkali metal salt include: an aromatic hydrocarbon-based solvent (including halogenated hydrocarbon), such as toluene, o-xylene, m-xylene, p-xylene, benzene, ethylbenzene, isopropylbenzene, 1,2,3-trimethylbenzene, 1,2,4-trimethylbenzene, 1,3,5-trimethylbenzene, tetralin, cymene, methylethylbenzene, 2-ethyltoluene, chlorobenzene and dichlorobenzene; a linear aliphatic hydrocarbon-based solvent(including halogenated hydrocarbon), such as pentane, hexane, heptane, octane, decane, dodecane, undecane, tridecane, decalin, 2,2,4,6,6-pentamethylheptane, isoparaffin (e.g., "MARUKASOL R" (a mixture of 2,2,4,6,6-pentamethylheptane and 2,2,4,4,6-pentamethylheptane, manufactured by Maruzen Petrochemical Co., Ltd.), "Isopar (registered trademark) G" (a C9-C11-mixed isoparaffin, manufactured by Exxon Mobil Corporation), "Isopar (registered trademark) E" (a C8-C10-mixed isoparaffin, manufactured by Exxon Mobil Corporation), dichloromethane, chloroform and 1,2-dichloroethane; a cyclic aliphatic hydrocarbon-based solvent, such as cyclohexane, methylcyclohexane, 1,2-dimethylcyclohexane, 1,3-dimethylcyclohexane, 1,4-dimethylcyclohexane, ethylcyclohexane, 1,2,4-trimethylcyclohexane, 1,3,5-trimethylcyclohexane, propylcyclohexane, butylcyclohexane and "SWACLEAN 150" (a C9-alkylcyclohexane mixture, manufactured by Maruzen Petrochemical Co., Ltd.); and an aromatic ether-based solvent, such as anisole, 2-methylanisole, 3-methylanisole and 4-methylanisole. These solvents may be used singly, or two or more of them may be used in the form of a mixture. Among these solvents, toluene, o-xylene, m-xylene, p-xylene, ethylbenzene, isopropylbenzene, 1,2,4-trimethylbenzene, hexane, heptane, chlorobenzene, dichlorobenzene, dichloromethane, 1,2-dichloroethane, anisole and cyclohexane are preferred. These solvents may be used singly, or two or more of them may be used.

Among these solvents, at least one kind selected from the group consisting of the aromatic hydrocarbon-based solvent (including halogenated hydrocarbon), the aliphatic hydrocarbon-based solvent (including halogenated hydrocarbon), and the aromatic ether-based solvent is preferred.

Considering an influence on a final product battery, disuse of water as the solvent for the above reaction and the below-mentioned purification is preferred.

The poor solvent alone or a mixed solvent of the poor solvent and the good solvent is preferably used for the reaction between bis(fluorosulfonyl)imide and the alkali metal halide. When using the mixed solvent, the ratio of the poor solvent in the total amount of the poor solvent and the good solvent is preferably 70% by weight or more, more preferably 80% by weight or more. Increasing a proportion of the poor solvent in the organic solvent as described above yields the precipitation of the bis (fluorosulfonyl) imide alkali metal salt as an insoluble solid in the reaction solution as explained before. As a result, the bis (fluorosulfonyl) imide alkali metal salt can be separated easily by filtering as a simple purification method without an operation for volatilizing the solvent in the reaction solvent.

As the poor solvent used for above reaction, toluene, o-xylene, m-xylene, p-xylene, ethylbenzene, isopropylbenzene, 1,2,4-trimethylbenzene, hexane, heptane, chlorobenzene, dichlorobenzene, dichloromethane, 1,2-dichloroethane, anisole and cyclohexane are preferred. The most preferable solvent is cyclohexane.

In the production method of the present invention, the content of the organic solvent to the total reaction solution is preferably 5 to 95% by weight and more preferably 5 to 90% by weight. The lower limit of the organic solvent content is more preferably 10% by weight or more, still more preferably 15% by weight or more, further preferably 20% by weight or more, particularly preferably 30% by weight or more and most preferably 50% by weight or more. The upper limit of the organic solvent content is more preferably 85% by weight or lower and still more preferably 80% by weight or lower. When the content of the organic solvent to the total reaction solution is included in the above range, the reaction proceeds to conduct purification step easily.

### [Reaction conditions]

The reaction temperature of the reaction between bis(fluorosulfonyl)imide and the alkali metal halide (the "reaction temperature" is, for examples, the temperature of the reaction solvent in the examples below) can set to 10 to 200°C, and furthermore 20 to 200°C. The upper limit of the reaction temperature is preferably 180°C or lower and more preferably 160°C or lower. The reaction temperature is not limited to above temperature range. Low reaction temperatures may reduce the reaction rate and high reaction temperatures may generate impurities, thus they are undesirable.

The pressure of the reaction between bis(fluorosulfonyl)imide and the alkali metal halide can be performed under high pressure, normal pressure or reduced pressure. The degree of the reaction pressure is preferably 1250hPa or lower, more preferably 1150hPa or lower, and still more preferably 1050hPa or lower. The lower limit of the reaction pressure can set to about 10hPa.

The order of addition of materials in the reaction between bis(fluorosulfonyl)imide and the alkali metal halide is not particularly limited. The reaction can be conducted while adding the alkali metal halide to the mixture of the organic solvent and bis(fluorosulfonyl)imide, or the reaction can be conducted while adding bis(fluorosulfonyl)imide to the mixture of the organic solvent and the alkali metal halide. Also, the reaction can be conducted while adding the mixture of the organic solvent and the alkali metal halide to the mixture of the organic solvent and bis(fluorosulfonyl)imide, or the reaction can be conducted while adding the mixture of the organic solvent and bis(fluorosulfonyl)imide to the mixture of the organic solvent and the alkali metal halide. It is also possible to initiate the reaction after mixing bis(fluorosulfonyl)imide, the alkali metal halide and the organic solvent.

The reaction time (i.e., mixing time in the reaction) can be set to, for example, 0.1 to 24 hours, preferably 0.5 to 12 hours and more preferably 1 to 8 hours.

In the above production method, a hydrogen halide as a volatile matter is generated as a by-product in the reaction solution. In the production method of the present invention, this generated hydrogen halide is preferably removed from the reaction solution during the reaction by a volatilization operation mentioned below as for example.

### [Volatilization operation]

The production method of the present invention preferably includes a volatilization operation by normal pressure, decompression and/or heating for removing the volatile matter in the reaction solution. The production method of the present invention preferably includes a volatilization operation for removing the volatile matter in the reaction solution by decompression and/or heating. The volatilization operation for removing the volatile matter in the reaction solution by normal pressure, decompression and/or heating can be conducted during the reaction or after the reaction. The volatilization operation during the reaction can remove the volatile matter such as hydrogen halide generated during the reaction described above and thereby the reaction between bis (fluorosulfonyl) imide and the alkali metal halide is promoted and the purification can be conducted efficiently.

In the reaction between bis(fluorosulfonyl)imide and the alkali metal halide, if the alkali metal halide is a fluoride, particularly LiF, a by-product is easily removed by the volatilization operation because HF which is a volatile matter (particularly hydrogen halide) generated as a by-product by the reaction between bis (fluorosulfonyl) imide and the alkali metal halide has a low boiling point. As a result, the purification of the bis (fluorosulfonyl) imide alkali metal salt is conducted easily. Furthermore, fluoride, particularly LiF, is preferred because it has a sufficiently smaller influence on final product batteries than chlorides. In addition, when the metal constituting the alkali metal halide is Li, a lithium ion secondary battery achieves excellent battery properties.

The volatilization operation is not particularly limited, and may be performed either under normal pressure or reduced pressure. From the viewpoint of avoiding the decomposition of the bis(fluorosulfonyl)imide alkali metal salt by heating, the volatilization operation is desirably performed under reduced pressure out of normal pressure, reduced pressure and heating. When conducting the volatilization under reduced pressure, a degree of reduction in pressure is not particularly limited, and can be adjusted appropriately depending on the types of the volatile matter, particularly depending on the types of the hydrogen halides. For example, the degree of reduction in pressure is preferably 100 kPa or less (1000hPa or less), more preferably 40 kPa or less (400hPa or less), still more preferably 15 kPa or less (150hPa or less) and most preferably 5 kPa or less (50hPa or less). The lower limit of the degree of reduction can be about 1 kPa (10hPa).

A volatilization temperature is not particularly limited, and can be adjusted appropriately depending on the degree of reduction in pressure, the types of the volatile matter and the types of the organic solvent. From the viewpoint of avoiding the decomposition of the bis(fluorosulfonyl) imide alkali metal salt by heat, the volatilization step is desirably performed at relatively low temperatures. For example, the volatilization temperatures are preferably 10 to 110°C, more preferably 15 to 80°C, still more preferably 20 to 60°C, particularly preferably 30 to 50°C.

A time for the volatilization is not particularly limited, and can be adjusted appropriately depending on the degree of reduction in pressure, the heating temperature, the amount of the volatile matter, the amount of the organic solvent and the like. For example, the time for the volatilization is preferably 0.1 to 24 hours, more preferably 0.5 to 12 hours, still more preferably 1 to 8 hours, particularly preferably 2 to 5 hours.

A device to be used for the volatilization step and capable of achieving the decompression and/or heating may be selected appropriately depending on the volume of the solution, the degree of reduction in pressure, the heating temperature and the like. For example, a tank-type reactor and a tank-type reactor which is capable of reducing an internal pressure can be mentioned. The volatilization operation can be conducted by using a different reactor from the reactor used for the reaction. From the view point of conveniences, the reactor used for the reaction is preferably used for the volatilization operation.

### [Purification step]

The production method of the present invention can include a purification step for conducting a filtering. Particularly, it is preferable to include a purification step for filtering the reaction solution obtained after the reaction. Filtering the reaction solution obtained after the reaction enables to obtain the bis(fluorosulfonyl) imide alkali metal salt as a solid matter such as filter residues from the solution obtained after the reaction. The purification step can includes, in addition to the filtering, publicly known operations such as solid precipitation e.g. crystallization; distillation; and concentration.

As a filtering method, pressure filtration and suction filtration are exemplified. The preferable conditions for the filtering is as follows: As the usable filter medium a filter made of, a fluororesin such as PTFE, a stainless steel fiber, polyolefin such as polyethylene, ultra high density polyethylene, nylon, a cellulose fiber, a glass fiber, a silica fiber, polycarbonate, cotton, polyethersulfone, cellulose acetate are exemplified. Among these examples, more preferable examples are a fluororesin, a stainless steel fiber, and polyethylene, and still more preferable examples are a fluororesin and a stainless steel fiber. The retained particle diameter of the filter medium is preferably 0.1 to 10 µm and more preferably 0.1 to 5 µm.

The filtering temperature (the temperature of the solution to be filtered after the reaction) is set to 0 to 70°C, preferably 0 to 50°C and more preferably 20 to 50°C.

Washing is preferably operated after the filtering. More preferably, the washing for the filter residue is operated after the filtering in the purification step for conducting the filtering by using a poor solvent for the bis(fluorosulfonyl) imide alkali metal salt. The poor solvent is preferably selected from the group consisting of the aromatic hydrocarbon solvent (including halogenated hydrocarbon), the aliphatic hydrocarbon solvent (including halogenated hydrocarbon) and the aromatic ether solvent. The washing enables to remove unreacted bis(fluorosulfonyl) imide sufficiently. As the solvent for the washing, the poor solvent having low affinity with bis(fluorosulfonyl) imide alkali metal salt is preferable to use. Specific examples of the solvent include: an aromatic hydrocarbon-based solvent (including halogenated hydrocarbon), such as toluene, o-xylene, m-xylene, p-xylene, benzene, ethylbenzene, isopropylbenzene, 1,2,3-trimethylbenzene, 1,2,4-trimethylbenzene, 1,3,5-trimethylbenzene, tetralin, cymene, methylethylbenzene, 2-ethyltoluene, chlorobenzene and dichlorobenzene; a linear aliphatic hydrocarbon-based solvent(including halogenated hydrocarbon), such as pentane, hexane, heptane, octane, decane, dodecane, undecane, tridecane, decalin, 2,2,4,6,6-pentamethylheptane, isoparaffin (e.g., "MARUKASOL R" (a mixture of 2,2,4,6,6-pentamethylheptane and 2,2,4,4,6-pentamethylheptane, manufactured by Maruzen Petrochemical Co., Ltd.), "Isopar (registered trademark) G" (a C9-C11-mixed isoparaffin, manufactured by Exxon Mobil Corporation), "Isopar (registered trademark) E" (a C8-C10-mixed isoparaffin, manufactured by Exxon Mobil Corporation), dichloromethane, chloroform and 1,2-dichloroethane; a cyclic aliphatic hydrocarbon-based solvent, such as cyclohexane, methylcyclohexane, 1,2-dimethylcyclohexane, 1,3-dimethylcyclohexane, 1,4-dimethylcyclohexane, ethylcyclohexane, 1,2,4-trimethylcyclohexane, 1,3,5-trimethylcyclohexane, propylcyclohexane, butylcyclohexane and "SWACLEAN 150" (a C9-alkylcyclohexane mixture, manufactured by Maruzen Petrochemical Co., Ltd.); and an aromatic ether-based solvent, such as anisole, 2-methylanisole, 3-methylanisole and 4-methylanisole. These solvents may be used singly, or two or more of them may be used in the form of a mixture. The solvent having lower boiling point is preferred when operating a drying mentioned below. Preferable solvent includes; toluene, o-xylene, m-xylene, p-xylene, ethylbenzene, isopropylbenzene, 1,2,4-trimethylbenzene, hexane, heptane, chlorobenzene, dichlorobenzene, dichloromethane, 1,2-dichloroethane, anisole and cyclohexane. More preferable solvent includes; toluene, hexane, cyclohexane, heptane, dichloromethane and 1,2-dichloroethane. Particularly preferable solvent includes; cyclohexane and 1,2-dichloroethane. These solvents may be used singly, or two or more of them may be used.

An organic solvent (additional organic solvent) other than the organic solvent contained in the solution after the reaction can be added before and/or after the purification step for conducting the filtering. The poor solvent and the good solvent mentioned above is usable as the additional organic solvent. When the good solvent as the additional organic solvent is added before the purification step for conducting the filtering, the proportion of the poor solvent in the organic solvent after adding the additional organic solvent can be, for example, 70% by weight or more. After the purification step for conducting the filtering, the additional organic solvent is usable for recovering the bis(fluorosulfonyl) imide alkali metal salt and for storing the bis(fluorosulfonyl) imide alkali metal salt in a dissolved state in the solvent. Also, a concentration operation by volatilization of the organic solvent contained in the reaction solution after the reaction can be performed by decompression and/or heating before and/or after the purification step for conducting the filtering.

### [Drying and powdering step]

A bis(fluorosulfonyl) imide alkali metal salt obtained by the purification step can be used as a product without any modification. The bis(fluorosulfonyl) imide alkali metal salt can be powdered (powdering and drying step) for improving stability during its storage and facilitating a product distribution. When a bis(fluorosulfonyl) imide alkali metal salt in a solid state is obtained in the purification step, thus obtained solid may be directly dried in a dryer or the solid may be dissolved in a solvent which can dissolve the bis(fluorosulfonyl) imide alkali metal salt, that is, dissolving the solid in the good solvent alone, or the mixed solvent of the good solvent and the poor solvent and thereafter subjecting to a drying and powdering step.

The drying and powdering method of the bis (fluorosulfonyl) imide alkali metal salt is not particularly limited, and following methods are exemplified:
(1) A method for drying and powdering the solid bis(fluorosulfonyl) imide alkali metal salt obtained by the purification step for conducting the filtering;
(2) A method for drying and powdering a solution in which the solid obtained by the purification step is dissolved. For example, drying and powdering a precipitated and separated bis(fluorosulfonyl) imide alkali metal salt obtained from a solution obtained by dissolving the filter residue obtained by the filtering in the good solvent alone or in the mixed solvent of the good solvent and the poor solvent without any modification or from a solution after allowing such solution to standing while being cooled to 30 °C or lower as needed.
(3) A method for powdering by drying an after separated filter residue obtained by filtering a bis(fluorosulfonyl) imide alkali metal salt precipitated solution obtained by adding a solvent to a solution in which the solid obtained in the purification step is dissolved.

The solvent usable in the above (3) is any solvent having difficulty to form a solvation with the bis(fluorosulfonyl) imide alkali metal salt. As specific examples of the solvent usable in the above (3) method include: an aromatic hydrocarbon-based solvent (including halogenated hydrocarbon), such as toluene, o-xylene, m-xylene, p-xylene, ethylbenzene, isopropylbenzene, 1,2,4-trimethylbenzene, 1,3,5-trimethylbenzene, 1,2,3-trimethylbenzene, chlorobenzene, dichlorobenzene and anisole; an aliphatic hydrocarbon-based solvent(including halogenated hydrocarbon), such as hexane, heptane, octane, nonane, decane, undecane, dodecane, decalin, dichloromethane, 1,2-dichloroethane and cyclohexane. Also, the solvent is preferably added in an amount of 20 mass times or less, more preferably 10 mass times or less against 1 mass part of a concentrate which is an almost saturated solution of the bis(fluorosulfonyl)imide alkali metal salt.

Subsequently, the precipitated bis(fluorosulfonyl)imide alkali metal salt is separated from the solution or the like by a gradient method, a centrifugal separation method, a filtering method and the like and then dried. The drying method of the bis(fluorosulfonyl)imide alkali metal salt is not particularly limited, and any one of the conventional known dryer can be employed. Drying under reduced pressure is preferably employed. The pressure of drying under reduced pressure is, for examples, 1000hPa or lower, more preferably 400hPa or lower, still more preferably 150hPa or lower and the lower limit of the pressure can be set to be about 10hPa. The drying temperature is preferably set to 0 to 100°C and more preferably 10°C or higher, still more preferably 20°C or higher and more preferably 80°C or lower, still more preferably 60°C or lower.

The drying time can be, for example, set to 1 to 48 hours depending on implementation scale and a drying method.

The drying of the bis(fluorosulfonyl)imide alkali metal salt can be carried out while supplying a gas to the dryer. Examples of the usable gas include the gas used in the purification step, and an inert gas such as nitrogen and argon; dry air are exemplified.

The solid / powder of the bis(fluorosulfonyl)imide alkali metal salt obtained by the above method may be subjected to further purification operation for further improving its purity as needed. As a purification operation, any conventionally known purification methods can be employed.

### [Recovery step]

The production method of the invention can include a recovery step for the bis(fluorosulfonyl)imide alkali metal salt, a compound having a sulfonylimide skeleton, a raw material, or a by-product separated from a product in each of the above steps. The yield of bis(fluorosulfonyl)imide alkali metal salt can be improved by recovering the bis(fluorosulfonyl)imide alkali metal salt remained particularly in a waste liquid discharged from the purification step such as filtering or in a solution (mother liquor) from which the bis(fluorosulfonyl)imide alkali metal salt precipitated in the powdering and drying step is removed.

When the purity of the bis(fluorosulfonyl)imide alkali metal salt obtained in the drying and powdering step is low, it may be further purified independently, but the bis(fluorosulfonyl)imide alkali metal salt in a solid state (powder) may be mixed with a recovery solution (the above waste solution or mother liquor). The operation in the drying and powdering step also corresponds to a purification operation such as crystallization and reprecipitation method, so that the bis(fluorosulfonyl)imide alkali metal salt from the waste liquid or mother liquor is recovered and the purity of the bis(fluorosulfonyl)imide alkali metal salt is improved.

The method for further purification of the recovered the bis(fluorosulfonyl)imide alkali metal salt is not particularly limited, and a solution recovered from each step may be purified independently or in combination to recover bis (fluorosulfonyl) imide alkali metal salt. And a recovered solution can be supplied to either the purification step, or the powdering and drying step. From the viewpoint of productivity, the recovered solution is preferably supplied to the purification step.

### [Electrolyte solution material containing the bis(fluorosulfonyl)imide alkali metal salt]

The bis (fluorosulfonyl) imide alkali metal salt obtained by the present invention is suitable for a non-aqueous electrolyte solution and can be used as an electrolyte solution material by being dissolved and diluted in a solvent for an electrolyte solution material. Also, a non-aqueous electrolyte solution can be produced from the electrolyte solution material without any modification or by merely diluting the electrolyte solution material.

Since the solvent for an electrolyte solution material is usable as the electrolyte solution material without any modifications, a solvent for an electrolyte solution material may be referred to as an electrolyte solvent in the present specification.

The solvent for an electrolyte solution material, that is, a electrolyte solution solvent is preferably at least one selected from the group consisting of a carbonate-based solvent, a cyclic ether-based solvent, a chain ether-based solvent having two or more of oxygen atoms within its molecule, a cyclic ester-based solvent, a sulfolane- based solvent, N,N-dimethylformamide, dimethyl sulfoxide and N-methyloxazolidinone. Specific examples include: a carbonate-based solvent, such as ethylene carbonate, propylene carbonate, butylene carbonate, dimethyl carbonate, ethylmethyl carbonate and diethyl carbonate; a linear ether-based solvent having two or more of oxygen atoms within its molecule, such as dimethoxymethane and 1,2-dimethoxyethane; a cyclic ether-based solvent, such as tetrahydrofuran, 2-methyltetrahydrofuran, 1,3-dioxane and 4-methyl-1,3-dioxolane; a cyclic ester-based solvent, such as γ-butyrolactone and γ-valerolactone; a sulfolane- based solvent, such as sulfolane and 3-methylsulfolane; and N,N-dimethylformamide, dimethyl sulfoxide and N-methyloxazolidinone. These solvents may be used singly, or two or more of them may be used in the form of a mixture. Among these exemplified solvents, the carbonate-based solvent such as ethylene carbonate, propylene carbonate, butylene carbonate, dimethyl carbonate, ethylmethyl carbonate and the diethyl carbonate (particularly a cyclic carbonate such as ethylene carbonate, propylene carbonate and butylene carbonate) and the cyclic ester-based solvent such as γ-butyrolactone and γ-valerolactone are preferred and the carbonate-based solvent is particularly preferred.

The concentration of the bis(fluorosulfonyl)imide alkali metal salt to be contained in the electrolyte solution material is not limited particularly, and can be adjusted appropriately depending on the types of the electrolyte solvents. For example, the concentration of the bis(fluorosulfonyl)imide alkali metal salt is preferably 15 to 95% by mass, more preferably 20 to 90% by mass, still more preferably 30 to 90% by mass. In the production of a non-aqueous electrolyte solution by adding the organic solvent to the electrolyte solution material, from the viewpoint of appropriately setting the concentration of the electrolyte salt in the non-aqueous electrolyte solution, the concentration of the bis(fluorosulfonyl)imide alkali metal salt to be contained in the electrolyte solution material is preferably 30% by mass or more, more preferably 40% by mass or more, still more preferably 50% by mass or more. When the electrolyte solution material according to the present invention contains the bis(fluorosulfonyl)imide alkali metal salt at a concentration of 30% by mass or more, good stability of the bis(fluorosulfonyl)imide alkali metal salt can be achieved and the generation of HF (hydrofluoric acid), which can cause the corrosion of a container for storage or transport use, can be prevented, and therefore this concentration is also suitable for the storage and transportation of the bis(fluorosulfonyl)imide alkali metal salt.

The electrolyte solution material containing the bis(fluorosulfonyl)imide alkali metal salt produced by the production method according to the present invention can be used suitably as a material for an ionic conductor that constitutes a primary battery, a battery having a charge/discharge mechanism, such as a lithium ion secondary battery and a fuel cell or an electrical storage device (an electrochemical device) such as an electrolytic capacitor, an electric double-layer capacitor and a solar cell, and an electrochromic display element.

The present invention also includes, within the scope thereof; a non-aqueous electrolyte solution produced using the electrolyte solution material; and a method for producing a non-aqueous electrolyte solution using the electrolyte solution material. A non-aqueous electrolyte solution can be produced by mixing a non-aqueous electrolyte solution preparation solvent with the electrolyte solution material, if necessary. In the non-aqueous electrolyte solution, various types of electrolytes, additives and the like may be added for the purpose of improving battery properties. It is also possible to add a solvent suitable for the dissolution of an electrolyte or the like to the electrolyte solution material. In the preset invention, the non-aqueous electrolyte can be prepared by adding a desired solvent to the electrolyte solution material.

The electrolyte solution preparation solvent to be used is not particularly limited, as long as the solvent is compatible with the electrolyte solvent and can dissolve and disperse a desired electrolyte salt therein. In the present invention, any one of the conventional known solvents for batteries, such as a non-aqueous solvent and a medium (e.g., a polymer, a polymer gel) that can be used in place of the solvent, can be used. In the electrolyte solution material, the electrolyte solvent is contained. If required, the electrolyte solution material may additionally be added a solvent that is of the same type as the electrolyte solvent, and any one of the above-mentioned electrolyte solvents may be used as the solvent. The electrolyte solution preparation solvent may be in a liquid form or a solid form, and is preferably in a liquid form from the viewpoint of the achievement of highly efficient mixing. The temperature of the electrolyte solution preparation solvent is not particularly limited. The temperature may be room temperature, or may be adjusted appropriately as required.

The electrolyte solution preparation solvent is exemplified by an ether solvent such as ethylene glycol dimethyl ether, ethylene glycol diethyl ether, tetrahydrofuran, 2-methyltetrahydrofuran, 2,6-dimethyltetrahydrofuran, tetrahydropyran, crown ether, triethylene glycol dimethyl ether, tetraethylene glycol dimethyl ether, 1,4-dioxane and 1,3-dioxolan; a chain carbonate ester solvent such as dimethyl carbonate, ethyl methyl carbonate, diethyl carbonate, diphenyl carbonate and methyl phenyl carbonate; a cyclic carbonate solvent such as ethylene carbonate, propylene carbonate, 2,3-dimethylethylene carbonate, butylene carbonate, vinylene carbonate, 2-vinylethylene carbonate; an aromatic carboxylate ester solvent such as methyl benzoate and ethyl benzoate; a lactone solvent such as γ-butyrolactone, γ-valerolactone and δ-valerolactone; a phosphate ester solvent such as trimethyl phosphate, ethyl dimethyl phosphate, diethyl methyl phosphate and triethyl phosphate; a nitrile solvent such as acetonitrile, propionitrile, methoxypropionitrile, glutaronitrile, adiponitrile, 2-methylglutaronitrile, valeronitrile, butyronitrile and isobutyronitrile; a sulfur compound solvent such as dimethyl sulfone, ethyl methyl sulfone, diethyl sulfone, sulfolane, 3-methylsulfolane and 2,4-dimethylsulfolane; an aromatic nitrile solvent such as benzonitrile and tolunitrile; nitromethane, 1,3 -dimethyl-2-imidazolidinone, 1,3-dimethyl-3,4,5,6-tetrahydro-2(1H)-pyrimidinone, 3-methyl-2-oxazolidinone and the like.

Among the electrolyte solution preparation solvents, a carbonate ester (a carbonate-based solvent) such as a linear carbonate ester and a cyclic carbonate ester, a lactone and an ether are preferred; dimethyl carbonate, ethylmethyl carbonate, diethyl carbonate, ethylene carbonate, propylene carbonate, γ-butyrolactone, γ-valerolactone and the like are more preferred; and a carbonate-based solvent such as dimethyl carbonate, ethylmethyl carbonate, diethyl carbonate, ethylene carbonate and propylene carbonate is still more preferred. These solvents may be used singly, or two or more of them may be used in combination.

When the above-mentioned polymer or polymer gel is used in place of the solvent, the following method may be used. That is, a method in which a solution obtained by dissolving an electrolyte salt in the above-mentioned non-aqueous solvent is added dropwise to a polymer formed into a film by a publicly known method to impregnate the polymer with the electrolyte salt and the non-aqueous solvent or to support the electrolyte salt and the non-aqueous solvent; a method in which a polymer and an electrolyte salt are melted at a temperature of a melting point of the polymer or higher, mixed, and then formed into a film, and the film is impregnated with a non-aqueous solvent (these are gel electrolyte); a method in which a non-aqueous electrolytic solution obtained by dissolving an electrolyte salt in an organic solvent in advance is mixed with a polymer, and the resulting mixture is formed into a film by a casting method or a coating method, and an organic solvent is volatilized; and a method in which a polymer and an electrolyte salt are melted at a temperature of a melting point of the polymer or higher, mixed, and then molded (intrinsic polymer electrolyte) are exemplified.

Examples of the polymer, which is used in place of the medium, include polyether-based polymers such as polyethylene oxide (PEO) and polypropylene oxide which are a homopolymer or a copolymer of epoxy compounds (ethylene oxide, propylene oxide, butylene oxide, allyl glycidyl ether, etc.); methacrylic polymers such as polymethyl methacrylate (PMMA); nitrile-based polymers such as polyacrylonitrile (PAN); fluoropolymers such as polyvinylidene fluoride (PVdF) and polyvinylidene fluoride-hexafluoropropylene; and their copolymers.

In the present invention, if necessary, an electrolyte salt that is different from the bis(fluorosulfonyl)imide alkali metal salt (also referred to as "another electrolyte salt", hereinafter) may be mixed with the electrolyte solution material. Above-mentioned another electrolyte salt may be added to the electrolyte solution material to which the electrolyte solution preparation solvent is not added yet. From the viewpoint of the dissolution efficiency of above-mentioned another electrolyte salt, it is desirable to add above-mentioned another electrolyte salt after the addition of the electrolyte solution preparation solvent to the electrolyte solution material. For example, in the case where above-mentioned another electrolyte salt to be added is poorly soluble in ethylene carbonate, like LiPF₆, it is desirable to add the electrolyte salt after the addition of a solvent suitable for the dissolution of the electrolyte salt, as the electrolyte solution preparation solvent, to the electrolyte solution material.

Above-mentioned another electrolyte salt is not particularly limited, and may be any one of the conventional known electrolytes that may be used in electrolytes for lithium ion secondary batteries. As above-mentioned another electrolyte salt, such an electrolyte salt is exemplified by an inorganic cation salt and organic cation salt of trifluoromethanesulfonate ion (CF₃SO₃⁻), hexafluorophosphate ion (PF₆⁻), perchlorate ion (ClO₄⁻), tetrafluoroborate ion (BF₄⁻), hexafluoroarsenate ion (AsF₆⁻), tetracyanoborate ion ([B(CN)₄]⁻), tetrachloroaluminum ion (AlCl₄⁻), tricyanomethide ion (C[(CN)₃]⁻), dicyanamide ion (N[(CN)₂]⁻), tris(trifluoromethanesulfonyl)methide ion (C[(CF₃SO₂)₃]⁻), hexafluoroantimonate ion (SbF₆⁻) and dicyanotriazolate ion (DCTA) as an anion; a fluorosulfonylimide salt other than the bis(fluorosulfonyl)imide alkali metal salt. Specific examples include LiPF₆, LiPF₃(C₂F₅)₃, LiBF₄, LiBF(CF₃)₃, preferably LiPF₆ or LiBF₄, and more preferably LiPF₆. When the electrolyte solution preparation solvent and above-mentioned another electrolyte salt are mixed with the electrolyte solution material according to the present invention to produce the non-aqueous electrolyte solution, the generation of heat during the mixing of the electrolyte salt can be prevented, and therefore the decomposition of the non-aqueous electrolyte solution can be prevented, resulting in the production of the electrolyte solution having good quality.

When the non-aqueous electrolyte solution contains above-mentioned another electrolyte salt, the amount of another electrolyte salt is not particularly limited as long as the total concentration of above-mentioned another electrolyte salt and the bis(fluorosulfonyl)imide alkali metal salt is equal to a saturated concentration or lower. The content of above-mentioned another electrolyte salt is preferably 0.5 mol/L or more, more preferably 0.8 mol/L or more, still more preferably 1.0 mol/L or more and preferably 2.5 mol/L or less, more preferably 2.0 mol/L or less and still more preferably 1.5 mol/L or less.

The ratio between the bis(fluorosulfonyl)imide alkali metal salt and above-mentioned another electrolyte salt is not particularly limited. Therefore, the ratio between the bis(fluorosulfonyl)imide alkali metal salt and above-mentioned another electrolyte salt may be the same, or one of them may be higher.

The proportion of above-mentioned another electrolyte salt may be higher than the bis(fluorosulfonyl)imide alkali metal salt.

To obtain a further excellent resistance to short circuit prevention and an effect of improving the capacity retention rate (cycle properties) at the time of charging and discharging by increasing the concentration ratio of the bis(fluorosulfonyl)imide alkali metal salt, the preferable concentration ratio is bis (fluorosulfonyl) imide alkali Metal salt: above-mentioned another electrolyte salt = 1: 1 to 2: 1.

The non-aqueous electrolytic solution of the invention may contain an additive to improve various properties of the lithium ion secondary battery. The additive may be added at any stages in the process of manufacturing the non-aqueous electrolytic solution, and is not particularly limited and, for example, the additive may be added after the addition of the electrolyte salt.

The additive is exemplified by a cyclic carbonate having a unsaturated bond, such as vinylene carbonate (VC), vinylethylene carbonate (VEC), methylvinylene carbonate (MVC) and ethylvinylene carbonate (EVC); a carbonate compound such as fluoroethylene carbonate, trifluoropropylene carbonate, phenylethylene carbonate and erythritan carbonate; a carboxylic acid anhydride such as succinic anhydride, glutaric anhydride, maleic anhydride, citraconic anhydride, glutaconic anhydride, itaconic anhydride, diglycolic anhydride, cyclohexanedicarboxylic anhydride, cyclopentanetetracarboxylic dianhydride and phenylsuccinic anhydride; a sulfur-containing compound such as ethylene sulfite, 1,3-propanesultone, 1,4-butanesultone, methyl methanesulfonate, busulfan, sulfolane, sulfolene, dimethyl sulfone, tetramethylthiuram monosulfide and trimethylene glycol sulfate ester; a nitrogen-containing compound such as 1-methyl-2-pyrrolidinone, 1-methyl-2-piperidone, 3-methyl-2-oxazolidinone, 1,3-dimethyl-2-imidazolidinone and N-methylsuccinimide; a phosphate such as monofluorophosphate and difluorophosphate; a saturated hydrocarbon compound such as heptane, octane and cycloheptane.

The concentration of the above-described additive in 100% by mass of the non-aqueous electrolyte solution is preferably 0.1% by mass or more, more preferably 0.2% by mass or more, still more preferably 0.3% by mass or more and 10% by mass or less, more preferably 8% by mass or less and still more preferably 5% by mass or less. When the usage amount of the additive is too small, it may be possibly difficult to obtain an effect by the additive in some cases. Alternatively, even when a large amount of the additive is used, an effect commensurate with added amount may be hardly obtained and conductivity may be possibly decreased due to high viscosity of the non-aqueous electrolyte solution.

It should be noted that non-aqueous electrolyte solution 100% by mass means the sum of all the components contained in the non-aqueous electrolyte solution such as the above-mentioned bis(fluorosulfonyl)imide alkali metal salt, above-mentioned another electrolyte salt, the solvent, and optionally used additives.

The present application claims the benefit of the priority date of Japanese patent application No. 2016-106033 filed on May 27, 2016. All of the contents of the Japanese patent application No. 2016-106033 filed on May 27, 2016 are incorporated by reference herein.

Hereinafter, the present invention is described in detail with Examples. However, the present invention is not limited to the following Examples in any way, and it is possible to carry out the present invention according to the Examples with an additional appropriate change within the range of the above descriptions and the following descriptions. Such a change is also included in the technical scope of the present invention.

### Example 1

In a PFA (fluororesin) made reaction container, 1.17 g (45 mmol) of LiF and 20 g of dichloroethane were weighed and introduced. 9.05 g (50 mmol) of HFSI [bis(fluorosulfonyl)imide] was introduced into the reaction container. Thereafter, the reaction solution was stirred at 25° C under atmospheric pressure for 5 hours for reaction. After the reaction, a solution after the reaction in which LiFSI [bis(fluorosulfonyl)imide lithium salt] precipitated was obtained. The solution after the reaction was filtered under pressure using PTFE filter paper (retained particle diameter 1 µm), and the filtrate was washed with 10g of 1,2-dichloroethane. Thereafter, the obtained filter residue was dried under reduced pressure at 50°C under approximately 100 hPa for 12 hours to obtain 8.40 g (45 mmol) of LiFSI [bis (fluorosulfonyl) imide lithium salt].

### Example 2

In a PFA (fluororesin) made reaction container, 0.324 g (12.5 mmol) of LiF and 20 g of cyclohexane were weighed and introduced. 2.5 g (13.8 mmol) of HFSI [bis(fluorosulfonyl)imide] was introduced into the reaction container. Thereafter, the reaction solution was stirred at 25°C under atmospheric pressure for 1 hours. After the reaction, a solution after the reaction in which LiFSI [bis(fluorosulfonyl)imide lithium salt] precipitated was obtained. The solution after the reaction was filtered under pressure using PTFE filter paper (retained particle diameter 1 µm). The obtained filter residue was washed with 10g of cyclohexane and then dried under reduced pressure at 50°C under approximately 100 hPa for 12 hours to obtain 2.33 g (12.5 mmol) of LiFSI [bis(fluorosulfonyl)imide lithium salt].

### Comparative Example 1

In a PFA (fluororesin) made reaction container, 1.34 g (55 mmol) of LiF and 20 g of acetonitrile were weighed and introduced. 9.05 g (50 mmol) of HFSI [bis(fluorosulfonyl)imide] was introduced into the reaction container. Thereafter, the reaction solution was stirred at 25 °C under atmospheric pressure for 5 hours for reaction. A solution after the reaction was centrifuged to remove solids. Thus obtained solution was processed by an evaporation under reduced pressure at 50°C to obtain 9.35g (50mml) LiFSI [bis(fluorosulfonyl)imide lithium salt]. It was confirmed by gas chromatography that acetonitrile as impurities remained in the product.

### INDUSTRIAL APPLICABILITY

The bis(fluorosulfonyl)imide alkali metal salt produced by the production method according to the present invention can be used suitably as a material for an ionic conductor that constitutes a primary battery, a battery having a charge/discharge mechanism such as a lithium ion secondary battery and a fuel cell or an electrical storage device (an electrochemical device) such as an electrolytic capacitor, an electric double-layer capacitor, a solar cell and an electrochromic display element.

## Claims

1. A method for producing a bis(fluorosulfonyl)imide alkali metal salt, comprising:
a step for producing a bis(fluorosulfonyl)imide alkali metal salt by reacting a bis(fluorosulfonyl)imide with an alkali metal halide in a reaction solution including an organic solvent, and
a purification step for filtering the bis(fluorosulfonyl)imide alkali metal salt from the reaction solution after the reaction.

2. The method for producing the bis(fluorosulfonyl)imide alkali metal salt according to claim 1, wherein
the organic solvent includes a poor solvent for the bis(fluorosulfonyl)imide alkali metal salt, wherein
the poor solvent is at least one selected from the group consisting of an aromatic hydrocarbon-based solvent (including halogenated hydrocarbon), an alphatic hydrocarbon-based solvent (including halogenated hydrocarbon), and an aromatic ether-based solvent.

3. The method for producing the bis(fluorosulfonyl)imide alkali metal salt according to either claim 1 or 2, wherein
a ratio of the poor solvent in the organic solvent is 70% by weight or more.

4. The method for producing the bis(fluorosulfonyl)imide alkali metal salt according to any one of claims 1 to 3, wherein
the organic solvent is a poor solvent for the bis(fluorosulfonyl)imide alkali metal salt, wherein
the poor solvent is at least one selected from the group consisting of an aromatic hydrocarbon-based solvent (including halogenated hydrocarbon), an alphatic hydrocarbon-based solvent (including halogenated hydrocarbon), and an aromatic ether-based solvent.

5. The method for producing the bis(fluorosulfonyl)imide alkali metal salt according to any one of claims 1 to 4, wherein
the poor solvent is at least one selected from the group consisting of toluene, o-xylene, m-xylene, p-xylene, ethylbenzene, isopropylbenzene,
1,2,4-trimethylbenzene, hexane, heptane, chlorobenzene, dichlorobenzene, dichloromethane, 1,2-dichloroethane, anisole and cyclohexane.

6. The method for producing the bis(fluorosulfonyl)imide alkali metal salt according to any one of claims 1 to 5, wherein
a mole ratio of the alkali metal halide to the bis(fluorosulfonyl)imide is 1.00 or less.

7. The method for producing the bis(fluorosulfonyl)imide alkali metal salt according to any one of claims 1 to 6, wherein
a generated hydrogen halide is removed from the reaction solution during the reaction.

8. The method for producing the bis(fluorosulfonyl)imide alkali metal salt according to any one of claims 1 to 7, wherein
the alkali metal halide is LiF.

9. The method for producing the bis(fluorosulfonyl)imide alkali metal salt according to any one of claims 1 to 8, wherein
the filtering is performed by using a filter medium having retained particle diameter of 0.1 to 10µm.

10. The method for producing the bis(fluorosulfonyl)imide alkali metal salt according to any one of claims 1 to 9, wherein
a washing for a filter residue is preferably operated by using a poor solvent for the bis(fluorosulfonyl) imide alkali metal salt after the filtering in the purification step for conducting the filtering, wherein
the poor solvent is at least one selected from the group consisting of an aromatic hydrocarbon-based solvent (including halogenated hydrocarbon), an alphatic hydrocarbon-based solvent (including halogenated hydrocarbon), and an aromatic ether-based solvent.
